# EUROPÄISCHE PATENTSCHRIFT

(11) **EP 1 617 912 B1**
(45) Veröffentlichungstag und Bekanntmachung des Hinweises auf die Patenterteilung: **24.10.2007**
(21) Anmeldenummer: 04726103.7
(22) Anmeldetag: 07.04.2004
(51) Int. Cl.: A61Q 15/00, A61Q 17/04, A61Q 19/00, A61K 8/31

(54) **OLIGO-ALPHA-OLEFIN-HALTIGE KOSMETISCHE ZUSAMMENSETZUNG**
COSMETIC OLIGO-ALPHA-OLEFIN CONTAINING COMPOUND
COMPOSITION COSMETIQUE CONTENANT UNE OLIGO-ALPHA-OLEFINE

(30) Priorität: 16.04.2003 DE 10317781; 28.05.2003 DE 10324508; 07.08.2003 DE 10336172
(43) Veröffentlichungstag der Anmeldung: 25.01.2006
(73) Patentinhaber: Cognis IP Management GmbH, 40589 Düsseldorf (DE)
(72) Erfinder: SCHMID, Karl, Heinz, 40822 Mettmann (DE); WESTFECHTEL, Alfred, 40724 Hilden (DE); ANSMANN, Achim, 40699 Erkrath (DE); DIERKER, Markus, 40597 Düsseldorf (DE); BRÜNING, Stefan, Philadelphia, PA 19118 (US)
(86) Internationale Anmeldenummer: PCT/EP2004/003693
(87) Internationale Veröffentlichungsnummer: WO 2004/091555

(56) Entgegenhaltungen:
- EP-A- 1 103 249
- EP-A- 1 232 739
- US-A- 3 954 897
- US-A- 4 339 344
- US-A1- 2001 034 461

## Beschreibung

### Gebiet der Erfindung

Die Erfindung betrifft neuartige kosmetische Zusammensetzungen, die bestimmte verzweigte Oligo-α-Olefine enthalten, sowie die Verwendung dieser verzweigten Oligo-α-Olefine als Ölkörper in kosmetischen und pharmazeutischen Zubereitungen.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet. Zur Herstellung kosmetischer bzw. pharmazeutischer Zubereitungen werden eine Vielzahl von natürlichen und synthetischen Ölen, beispielsweise Mandel- oder Avocadoöl, Esteröle, Ether, Alkylcarbonate, Kohlenwasserstoffe sowie Silikonöle eingesetzt. Eine wesentliche Aufgabe der Ölkomponenten ist es, neben der pflegenden Wirkung, die in unmittelbarem Zusammenhang mit der Hautfettung steht, dem Konsumenten ein nichtklebriges, möglich rasch eintretendes und lang anhaltendes Gefühl der Hautglätte und -geschmeidigkeit zu vermitteln.

Das subjektive Empfinden auf der Haut kann mit dem physikochemischen Parameter der Spreitung der Ölkörper auf der Haut korreliert und objektiviert werden, wie dies von U. Zeidler in der Fachzeitschrift Fette, Seifen, Anstrichmittel 87,403 (1985) dargestellt wurde. Demnach lassen sich kosmetische Ölkörper in niedrigspreitende (< 300 mm²/10 min), mittelspreitende (>/=300 bis <1000 mm² /10 min.) und hochspreitende Öle (>1= 1000 mm² / 10 min) einteilen. Setzt man in einer vorgegebenen Formulierung als Ölkörper ein hochspreitendes Öl ein, so wird zwar sehr rasch das gewünschte Gefühl der Hautglättung erzielt, und es wird im Falle der Verwendung von Cyclomethiconen, z.B. Dow Corning 245 fluid (Dow Corning Corporation) oder Abil® B 8839 (Goldschmidt Chemical Coperation) zusätzlich ein vom Verbraucher erwünschtes samtiges Endgefühl gefunden. Dieses Erlebnis dauert jedoch nicht lange an, da durch die hohe Flüchtigkeit der zuletzt genannten Strukturen das ausgeprägte Glättegefühl und damit die Samtigkeit sehr rasch verschwindet und ein unangenehmes stumpfes Gefühl auf der Haut zurück bleibt.

Cyclomethicone haben jedoch gegenüber anderen Emollients auf Kohlenwasserstoffbasis wie sehr leichten Mineralölen, Polybutylenen (z. B. Arlamol^{®} HD, ICI), Ethylhexylcyclohexan (Cetiol^{®} S, Cognis Deutschland GmbH & Co. KG) den Vorteil, dass sie auf der Haut als sehr leicht empfunden werden. Es bestand daher ein Bedarf an Ölkörpem/Emollients auf Kohlenwasserstoffbasis, die die Vorteile der Cyclomethicone wie leichtes Hautgefühl und gute Spreiteigenschaften auf sich vereinigen, ohne die Nachteile der Cyclomethicone zu zeigen.

Aus der EP 1 232 739 oder EP 1 103 249 ist die Verwendung von Tetraisobutylen in kosmetischen Zusammensetzungen bekannt.

Aufgabe der vorliegenden Erfindung ist es, verbesserte hochspreitende Ölkörper und diese enthaltende Zubereitungen zur Verfügung zu stellen, die ein rasch eintretendes und länger anhaltendes Gefühl der Hautglätte vermitteln und über eine gute Hautverträglichkeit verfügen. Darüber hinaus soll der Ölkörper einfach und stabil in Emulsionen einzuarbeiten und hydrolyseunempfindlich gegenüber pH - Schwankungen sein und zu niedrigviskosen Zusammensetzungen führen, die ein sehr leichtes Hautgefühl vermitteln. Ein weiterer Aspekt der Aufgabe war es, Ölkörper für den Antitranspirant/Deo-Bereich zur Verfügung zu stellen, die vergleichsweise hydrolyseunempfindlich sind und in Gegenwart der adstringierenden Aluminium- und Zirkonium-Verbindungen stabile Formulierungen erlauben.

### Beschreibung der Erfindung

Gegenstand der Erfindung ist eine kosmetische Zusammensetzung enthaltend wenigstens ein verzweigtes Oligo-α-Olefin, dadurch gekennzeichnet, dass die Seitenketten an wenigstens einer Verzweigungsstelle Ethyl-, Propyl- oder längerkettige verzweigte Alkylreste sind, weiches dadurch erhältlich ist, dass
a) wenigstens ein verzweigtes α-Olefin mit 5 bis 18 Kohlenstoffatomen,
b) wenigstens ein lineares α-Olefin mit 4 bis 10 Koblenstoffatomen
c) ein Gemisch aus einem verzweigten α-Olefin mit 4 bis 18 Kohlenstoffatomen und einem linearen α-Olefin mit 3 bis 18 Kohlenstoffatomen oder
d) ein Gemisch aus verschiedenen, verzweigten α-Olefinen mit 4 bis 18 Kohlenstoffatomen und linearen α-Olefinen mit 3 bis 18 Kohlenstoffatomen
in Gegenwart eines Katalysators - ausgewählt aus der Gruppe der organischen Säuren, der kationischen lonenenaustauscher, Kieselgele, Schichtsilikate, anorganischen Säuren oder Lewis-Säure basierten Katalysatoren - oligomerisiert wird.

Die spezifischen Oligo-α-Olefine zeichnen sich durch ein besonders hohes Spreitvermögen, eine verbesserte Sensorik der kosmetischen Endformulierung sowie eine besonders gute Stabilität in Antitranspirant/Deo-Formulierungen aus.

Erfindungsgemäß werden unter dem Begriff der Oligo-α-Olefine Dimere, Trimere, Tetramere, Pentamere und Hexamere der α-Oleflne verstanden, wobei der Einsatz von Dimeren, Trimeren, Tetrameren und Pentameren bevorzugt ist. Die Bedingungen für die Oligomerisierung und anschließende Hydrierung sind dem Fachmann hinlänglich bekannt. Die Herstellung von Oligomeren unter Verwendung von BF₃-haltigen Katalysatoren oder mit Metallocenkatalysatoren ist beispielsweise beschrieben in der WO 98/20053**,** DE-27 026 04**,** US-A 5,068,490**,** US-A 5,286,823 oder WO85101942. Die Reaktionsbedingungen werden je nach gewünschtem Oligomerisierungsgrad und gewünschter Jodzahl gewählt und während der Reaktion durch die üblichen, dem Fachmann bekannten Analysemethoden überprüft und eingestellt.

Die verzweigten, spezifischen Oligo-a-Olefine, die in der erfindungemäßen kosmetischen Zusammensetzung zum Einsatz kommen, sind geruchlose, farblose oder gelbliche Produkte, die - je nach Kettenlänge - flüssig oder fest sein können. Eine exakte Strukturformel für die Oligo-α-Olefine kann nicht angegeben werden, da bei der Oligomerisierung grundsätzlich Produktgemische entstehen, deren Fraktionen beispielsweise destillativ getrennt werden können.

Als organische Säure-Katalysatoren können z.B. para-Toluolsulfonsäure, Alkylbenzolsulfonsäure, Methansulfonsäure eingesetzt werden oder C2-C4-Mono-, Di- oder Tricarbonsäuren, wie z.B. Sulfobemsteinsäure. Als kationischer lonenaustauscher ist Lewatit® SPC 112 (Bayer AG) geeignet. Zu den Lewis-Säure basierten Katalysatoren zählen beispielsweise Borhalogenide, Aluminiumhalogenide oder Aluminiumalkylhalogenide.

Bevorzugt sind kosmetische Zusammensetzungen, in denen das verzweigte Oligo-α-Olefin 12 bis 36, vorzugsweise 12 bis 24 und insbesondere 14 bis 24 Kohlenstoffatomen aufweist Kosmetische Zusammensetzungen auf Basis verzweigter Oligo-α-Olefin 16 bis 20 Kohlenstoffatomen sind ganz besonders bevorzugt. In einer weiteren bevorzugten Ausführungsform der Erfindung werden die aus der Oligomerisierung resultierenden, verzweigten Oligo-α-Olefine anschließend hydriert wird. Die Hydrierung (Härtung) kann an dem technischen Produktgemisch vorgenommen werden, das direkt aus der Oligomerisierung resultiert. Sie kann aber auch nach destillativer Trennung der Fraktionen durchgeführt werden. Der zusätzliche Hydrierungsschritt (Härtung) führt zu Produkten mit erhöhter Oxidationsbeständigkeit. Mögliche Hydrierungsbedingungen sind z. B. in der internationalen Anmeldung PCT/EP02/11392 beschrieben. Als Katalysatoren eignen sich die aus dem Stand der Technik bekannten Hydrierkatalysatoren wie Nickel oder die Edelmetallkatalysatoren, insbesondere auf Basis von Platin oder Palladium. Als besonders geeignet Edelmetallkatalysatoren haben sich Palladiumkatalysatoren erwiesen, insbesondere Palladium auf Kohle.

Unter den Produkten, die durch Oligomerisierung der unter b) genannten linearen α-Olefine mit 4 bis 10 Kohlenstoffatomen erhalten werden, sind Tetramere bevorzugt. Besonders bevorzugt geeignet sind Tetramere des 1-Buten und des 2-Buten. Erfindungsgemäß bevorzugt ist auch der Einsatz von Oligo-α-Olefinen, wie sie üblicherweise bei der Polymerisation von 1-Buten oder Gemischen von 1-Buten und 2-Buten bei der Herstellung von Isotridecylalkoholen als Nebenprodukt entstehen und die aus dem Sumpfprodukt isoliert werden können. Ein großtechnisch ausgeübtes Verfahren ist z.B. das Octol-Verfahren der Firmen UOP und Degussa-Hüls AG, in dem bei Temperaturen von 30°C bis 250°C und Drücken von 20 bis 80 bar C3- und C4-Olefine an einem Katalysator, beispielsweise auf SiO₂ aufgebrachte Phosphorsäure, zu höheren Olefinen mit hohem Verzweigungsgrad umgesetzt werden. Dieses Verfahren ist u.a. in Petrochemical Processes, Band 1, Editions Technip (1989), S. 183-187 **und** In Hydrocarbon Processing, Februar 1992, Seite 45-46 beschrieben.

In einer weiteren bevorzugten Ausführungsform enthält die kosmetische Zusammensetzung verzweigte Oilgo-α-Olefine, die dadurch erhalten werden, dass ein Gemisch aus einem verzweigten α-Oiefin mit 5 bis 12 Kohlenstoffatomen und einem linearen α-Olefin mit 3 bis 12 Kohlenstoffatomen in Gegenwart eines Katalysators - ausgewählt aus der Gruppe der organischen Säuren, der kationischen lonenaustauscher, Meselgele, Schichtsilikate, anorganischen Säuren oder Lewis-Säure basierten Katalysatoren - oligomerisiert und ggf. anscbließend hydried wird.

Bevorzugte, lineare α-Olefine sind ausgewählt aus der Gruppe 1-Propen, 1-Buten, 2-Buten, 1-Penten und 2-Penten. Bevorzugte, verzweigte α-Olefine sind ausgewählt aus der Gruppe 2-Ethyl-1-hexen, 2-Propylhepten, 2-Methyl-1-buten, 2-Methyl-1-penten, 3-Methyl-1-penten, 4-Methyl-1penten.

Eine weitere bevorzugte Ausführungsform der kosmetischen Zusammensetzung enthält verzweigte Ollgo-α-Olefine, die dadurch erhältlich sind, dass ein Gemisch aus 80 %. Buten und 20 % iso-Buten in Gegenwart eines Katalysators - ausgewählt aus der Gruppe der organischen Säuren, der kationischen Ionenaustauscher, Kieselgele, Schichtsilikate, anorganischen Säuren oder Lewis-Säure basierten Katalysatoren - oligomerisiert und ggf. anschließend hydriert wird.

Die spezifischen, verzweigten Ollgo-α-Olefine eignen sich ausgezeichnet zur Verwendung als Ölkörper in kosmetischen oder pharmazeutischen Zubereitungen. Ein weiterer Gegenstand der Erfindung ist daher die Verwendung wenigstens eines Oligo-a-Olefins, weiches dadurch erhältlich ist, dass man
a) wenigstens ein verzweigtes α-Olefin mit 5 bis 18 Kohlenstoffatomen,
b) wenigstens ein lineares α-Olefin mit 4 bis 10 Kohlenstoffatomen
c) ein Gemisch aus einem verzweigten α-Olefin mit 4 bis 18 Kohlenstoffatomen und einem linearen α-Olefin mit 3 bis 18 Kohlenstoffatomen oder
d) ein Gemisch aus verschiedenen, verzweigten α-Olefinen mit 4 bis 18 Kohlenstoffatomen und linearen α-Olefinen mit 3 bis 18 Kohlenstoffatomen

in Gegenwart eines Katalysators - ausgewählt aus der Gruppe der organischen Säuren, der kationischen Ionenaustauscher, Kieselgele, Schichtsilikate, anorganischen Säuren oder Lewis-Säure basierten Katalysatoren - oligomerisiert und anschließend hydriert, als Ölkörper in kosmetischen oder pharmazeutischen Zubereitungen, insbesondere in Antitranspirant undloder Deo-Formulierungen. Der Einsatz dieser Oligo-α-Olefine in Antitranspirant-Formulierungen auf Basis adstringierender Aluminium- und insbesondere Aluminium/Zirkonium-Komplexe liefert besonders lagerstabile und hydrolyseunempfindliche Zusammensetzungen.

### Kosmetische Zubereitungen

Die erfindungsgemäße Verbindung erlaubt die Herstellung stabiler kosmetischer Emulsionen. Vorzugsweise handelt es sich hierbei um Formulierungen zur Körperpflege, z. B. in Form von Cremes, Milch, Lotionen, sprühbaren Emulsionen, Produkten zur Eliminierung des Körpergeruchs etc. Die erfindungsgemäßen Verbindung lässt sich auch in tensidhaltigen Formulleryngen wie z. B. Schaum- und Duschbädern, Haarshampoos und Pflegespülungen einsetzen.

Die kosmetischen Mittel können in Form von Emulsionen oder Dispersionen vorliegen, die Wasser und Ölphase nebeneinander enthalten. Bevorzugte kosmetische Zusammensetzungen sind solche in Form einer W/O- oder O/W-Emulsion mit den üblichen, dem Fachmann geläufigen, Konzentrationen an Ölen/ Fetten/Wachsen, Emulgatoren, Wasser und den in der Kosmetik üblichen, weiteren Hilfs- und Zusatzstoffen.

Zweckmäßig enthält die erfindungsgemäße kosmetische Zusammensetzung 1 bis 50 Gew.-%, bevorzugt 5 bis 40 Gew.-% und insbesondere 5 bis 25 Gew.-%, Ölkörper, die zusammen mit z.B. öllöslichen Tensiden/Emulgatoren und öllöslichen Wirkstoffen Bestandteil der sogenannten Öl- oder Fettphase sind. Zu den Ölkörpem werden im Sinne der Erfindung Fettstoffe, Wachse, und flüssige Öle gerechnet, nicht aber EmuigatorenlTenside. Die Poly-α-Olefine können als einziger Ölkörper oder aber in Kombination mit anderen Ölen/Fetten/Wachsen enthalten sein. Bevorzugt liegt der Anteil des wenigstens einen Oligo-α-Olefins bezogen auf die Gesamtmenge der Ölkörper bei 0,1 bis 100 Gew.-% und bevorzugt bei 1 bis 50 Gew.-%. Mengen von 1 bis 20 Gew.-% sind besonders bevorzugt.

Je nach Applikationszweck enthalten die kosmetischen Formulierungen eine Reihe weiterer Hilfs- und Zusatzstoffe wie beispielsweise oberflächenaktive Substanzen (Tenside, Emulgatoren), weitere Ölkörper, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., von denen einige nachstehend exemplarisch aufgelistet sind.

Die Mengen der jeweiligen Zusätze richten sich nach der beabsichtigten Verwendung.

### Oberflächenaktive Substanzen

In einer weiteren bevorzugten Ausführungsform enthält die kosmetische. Zusammensetzung 0,1 bis 20 Gew.-%, vorzugsweise 1 -15 Gew.-% und insbesondere 1 -10 Gew.-% einer oberflächenaktiven Substanz oder eines Gemisches aus oberflächenaktiven Substanzen.
Als oberflächenaktive Stoffe können anionische, nichtionische, kationische und/oder amphotere bzw. zwitterionische Tenside bzw. Emulgatoren oder ein beliebiges Gemisch dieser Tenside/Emulgatoren enthalten sein. In tensidhaitigen kosmetischen Zubereitungen, wie beispielsweise Duschgelen, Schaumbädern, Shampoos etc. ist vorzugsweise wenigstens ein anionisches Tensid enthalten, in Cremes und Lotionen für die Körperpflege sind vorzugsweise nicht-ionische Tenside/Emulgatoren enthalten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside/Emulgatoren** sind Fettalkoholpolyglycolether, Polyglycerinester, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside bzw. Glucoronsäurederivate - gegebenenfalls partiell oxidiert, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische. Tenside** sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel wie Cremes, Lotionen und Milch enthalten üblicherweise eine Reihe weiterer Ölkörper und Emollients, die dazu beitragen, die sensorischen Eigenschaften weiter zu optimieren. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyfistat, Isostearylpalmitat, Isostearylstearat, Isostearylisostearat, Isostearyloleat, Isostearyibehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol und Isopropanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dlcarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylylcarbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv^{®} TN), lineare oder verzweigte, symmetrische oder unsymmetrische Dialkylether mit 6 bis 22 Kohlenstoffatomen pro Alkylgruppe, wie z. B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) undloder aliphatische bzw. naphthenische Kohlenwasserstoffe, wie z. B. Mineralöl, Vaseline, Petrolatum, lsohexadecane, Squalan, Squalen oder Dialkylcyclohexane in Betracht.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste oder flüssige pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Giycerinmono/diiaurat, -palmitat oder-stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwächse und Polyethylenglycolwachse in Frage.

Als Periglanzwachse kommen beispielsweise in Frage: Alkylenglycolester, speziell Ethylenglycoldistearat; Fettsäurealkanolämide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Verdickungsmittel

Geeignete Verdickungsmittel sind beispielsweise Aerosll-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyacrylate, (z. B. Carbopole^{®} und Pemulen-Typen von Goodrich; Synthalene^{®} von Sigma;-Keltroi-Typen von Kelco; Sepiget-Typen von Seppic; Salcare-Typen von Allied Colloids), Polyacrylamide, Polymere, Polyvinylalkohol und Polyvinylpyrrolidon. Als besonders Wirkungsvoll haben sich auch Bentonite, wie z. B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Elektrolyte wie Kochsalz und Ammoniumchlorid.

### Stabillsatoren

Als Stabilisatoren können Metallsalze von Fettsäuren, wie z. B. Magnesium-, Aluminium- und/oder Zinkstearat bzw. -ricinoleat eingesetzt werden.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen sind z. B. zu nennen:
➢ 3-Benzylidencampher bzw. 3-Benzylidennorcampher und dessen Derivate, z. B. 3-(4-Methylbenzyliden)campher
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester
➢ Ester der Zimtsäure, vorzugsweise 4-Methoxyzimtsäure-2-ethythexylester, 4-Methoxyzimtsäurepropylester, 4-Methoxyzimtsäureisoamylester 2-Cyano-3,3-phenylzimtsäure-2-ethylhexylester (Octocrylene)
➢ Ester der Salicylsäure, vorzugsweise Salicylsäure-2-ethylhexylester, Salicylsäure-4-iso-propylbenzylester, Salioylsäurehomomenthylester
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
➢ Ester der Benzalmalonsäure, vorzugsweise 4-Methoxybenzmalonsäuredi-2-ethylhexyl-ester
➢ Triazinderivate, wie z. B. 2,4,6-Trianilino-(p-carbo-2'-ethyl-1'-hexyloxy)-1,3,5-triazin und Octyl-Triazon oder Dioctyl Butamido Triazone (Uvasorb® HEB)
➢ Propan-1,3-dione, wie z. B. 1-(4-tert.Butylphenyl)-3-(4'methoxyphenyl)propan-1,3-dion
➢ Ketotricyclo(5.2.1.0)decan-Derivate

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze sowie 2,2-(1,4-Phenylene)bis-1H-benzimidazole-4,6-disulfonsäure und deren Salze, insbesondere das Natrium-Salz,
➢ Sulfonsäurederivate von Benzophenonen, vorzugsweise 2-Hydroxy-4-methoxybenzo-phenon-5-sulfonsäure und ihre Salze
➢ Sulfonsäurederivate des 3-Benzylidencamphers, wie z. B. 4-(2-Oxo-3-bomylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 1-Phenyl-3-(4'-isopropylphenyl)-propan-1,3-dion sowie Enaminverbindungen. Die UV-A und UV-B-Fliter können selbstverständlich auch in Mischungen eingesetzt werden. Besonders günstige Kombinationen bestehen aus den Derivate des Benzoylmethans, z. B. 4-tert-Butyl-4'-methoxydibenzoylmethan (Parsol^{®} 1789) und 2-Cyano-3,3-phenylzimtsäure-2-ethyl-hexylester (Octo-crylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Uchtschutzpigmente, nämlich feindisperse Metalloxide bzw. Salze in Frage. Beispiele für geeignete Metalloxide sind Insbesondere Zinkoxid und Titandioxid. Als Salze können Silicate (Talk), Bariumsulfat oder Zinkstearat eingesetzt werden. Die Oxide und Salze werden in Form der Pigmente für hautpflegende und hautschützende Emulsionen verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Desodorierende Wirkstoffe

Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlor phenyl)hamstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen.

### Anitranspirante Wirkstoffe

Antitranspirant-Wirkstoffe reduzieren durch Beeinflussung der Aktivität der ekkrinen Schweißdrüsen die Schweißbildung, und wirken somit Achselnässe und Körpergeruch entgegen. Aufgrund ihrer Hydrolysestabilität und Kompatibilität mit Antitranspirant-Wirkstoffen sind die in Anspruch 1 genannten, spezifischen Oligo-α-Olefine besonders gut für den Antitranspirant-Sektor geeignet. Eine weitere bevorzugte Ausführungsform sind daher kosmetische Zusammensetzungen, dadurch gekennzeichnet, dass sie zusätzlich wenigstens einen antitranspiranten und/oder Deo-Wirkstoff enthält, vorzugsweise ein Aluminium-Zirkonium-Salz.

Als adstringierende Antitranspirant-Wirkstoffe eignen sich vor allem Salze des Aluminiums, Zirkoniums oder des Zinks. Solche geeigneten antihydmtisch wirksamen Wirkstoffe sind z. B. Aluminiumchlorid, Aluminiumchlofiydrat, Aluminiumdichlorhydrat, Aluminiumsesquichlofiydrat und deren Komplexverbindungen z. B. mit Propylenglycol-1,2, Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol^{®}, (4-Acetyl-1-{-4-[2-(2,4-dichlorphenyl) r-2-(1H-irnidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwefelteer Destillate, Salicylsäure (bzw. in Kombination mit Hexachlorophen), Undexylensäure Monoethanolamid Sulfosuccinat Na-Salz, Lamepon^{®} UD (Protein-Undecyiensäurekondensat), Zinkpyrithion, Aluminiumpyrithion und Magnesiumpyrithion / Dipyrithion-Magnesiumsulfat In Frage.

### Insekten-Repellentien

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

### Selbstbräuner und Depigmentlerungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, lso-propylalkohol, oder Polyole eingesetzt werden. Polyole, die hier in Betracht kommen, besitzen vorzugsweise 2 bis 15 Kohlenstoffatome und mindestens zwei Hydroxylgruppen.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

### Farbstoffe

Als Farbstoffe können die für kosmetische Zwecke geeigneten und zugelassenen Substanzen verwendet werden. Beispiele sind Kochenillerot A (C.l. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

### Beispiele

### Beispiel 1

560 g 2-Ethyl-1-hexen wird mit Lewatit® SPC 112 (Bayer AG) bei 100 °C und unter 10 bar Druck 3 Stunden lang oligomerisiert. 380 g des resultierenden Oligo-α-Olefins werden mit 0,05 % Palladium auf Kohle bei 200 °C 12 Stunden mit 100 bar Wasserstoff hydriert.

### Beispiel 2

700 g 2-Propyl-1-hepten wird mit Lewatit® SPC 112 (Bayer AG) bei 100 °C und unter 10 bar Druck 3 Stunden lang oligomerisiert. 380 g des resultierenden Oligo-α-Oleflns werden mit 0,05 % Palladium auf Kohle bei 200 °C 12 Stunden mit 100 bar Wasserstoff hydriert.

### Beispiel 3

500 g eines Olefingemisches (90 Gew.-% iso-Buten und 10 Gew.-% 1-Penten) wird mit Lewaöt® SPC 112 (Bayer AG) bei 100°C und unter 10 bar Druck 3 Stunden lang oligomerisiert. 380 g des oligomeren α-Olefins werden mit 0,05 % Palladium auf Kohle bei 200 °C 12 Stunden mit 100 bar Wasserstoff hydriert.

### Beispiel 4

500 g eines Olefingemisches (80 Gew.-% iso-Buten und 20 Gew.-% 1-Penten) wird mit Lewatit® SPC 112 (Bayer AG) bei 100 °C und unter 10 bar Druck 3 Stunden lang oligomerisiert. 380 g des oligomeren α-Olefins werden mit 0,05 % Palladium auf Kohle bei 200 °C 12 Stunden mit 100 bar Wasserstoff hydriert.

### Beispiel 5

500 g eines Oleflngemisches (80 Gew.-% iso-Buten und 20 Gew.-% 1-Buten) wird mit Lewatit® SPC 112 (Bayer AG) bei 100 °C und unter 10 bar Druck 3 Stunden lang oligomerisiert. 380 g des oligomeren α-Olefins werden mit 0,05 % Palladium auf Kohle bei 200 °C 12 Stunden mit 100 bar Wasserstoff hydriert.

### Beispiel 6

Ein Oligo-1-Buten-1 wird aus 1-Buten entsprechend der WO 98/20053 erhalten. Aus dem Oligomerengemisch wird durch Fraktionierung das Tetramer erhalten.

Hierzu wird Triisobutylaluminoxan nach der Vorschrift in EP-A 575 356 hergestellt. 35 g einer Lösung von Isobutylaluminoxan in Heptan (3 Gew.-% bezogen auf Al; 38,9 mmol Al), 2,7 g Trimethylaluminium und 180 g 1-Buten werden sukzessive in einem Reaktionsgefäß unter Inertgas vorgelegt und mit festem Biscyclopentadienylzirkon(IV)chlorid (3,2 g) versetzt. Man erwärmt ca. 22 h auf 50°C und gibt dann 10%ige Salzsäure unter Eiskühlung zu. Die organische Phase wird abgetrennt, das Lösungsmittel abdestilliert und das Oligomerengemisch durch Fraktionierung in Trimere, Tetramere, Pentamere und Hexamere aufgetrennt. Das Tetramer wurde entsprechend der Beispiele 1- 5 hydriert.

### Beispiel 7

Ein Oligo-1-Penten wird aus 1-Penten in Analogie zur Vorschrift in Beispiel 6 erhalten. Aus dem Oligomerengemisch wird durch Fraktionierung das Trimer gewonnen.

Hierzu wird Triisobutylaluminoxan nach der Vorschrift in EP-A 575 356 hergestellt. 35 g einer Lösung von Isobutylaluminoxan in Heptan (3 Gew.-% bezogen auf Al; 38,9 mmol Al), 2,7 g Trimethylaluminium und 200 g 1-Penten werden sukzessive in einem Reaktionsgefäß unter Inertgas vorgelegt und mit festem Biscyclopentadienylzirkon(IV)chlorid (3,2 g) versetzt. Man erwärmt ca. 22 h auf 50°C und gibt dann 10%ige Salzsäure unter Eiskühlung zu. Die organische Phase wird abgetrennt, das Lösungsmittel abdestilliert und das Oligomerengemisch fraktioniert. Das Trimer wurde entsprechend der Beispiele 1- 5 hydriert.

### Beispiel 8

560 g 2-Ethyl-1-hexen wird mit Lewatit SPC 112 (Bayer AG) bei 100 °C und unter 10 bar Druck 3 Stunden lang oligomerisiert. Es werden 380 g eines Gemisches erhalten, das mit 0,05 % Palladium auf Kohle bei 200 °C 12 Stunden mit 100 bar Wasserstoff hydriert wird. Das erhaltene Gemisch nach der Hydrierung besteht aus aus 75 Gew.-% Dimer (= Isohexadecan = C 16), 20 Gew.-% Trimer (= Iso-Tetracosan = C24) und 5 Gew.-% Tetramer (= Iso-C32-Kohlenwasserstoff).

### Kosmetische Zusammensetzungen

Mit den Oligo-a-Olefinen gemäß Beispiel 1 als Ölkomponente lassen sich folgende Emulsionen herstellen:

### Beispiel 8: O/W-Emulsion

| | | |
|---|---|---|
| Eumulgin^{®} B2 | 2 | Gew.-% |
| Lanette^{®} O | 5 | Gew.-% |
| Oligo-α-Olefin | 16 | Gew.-% |
| Glycerin | 3 | Gew.-% |
| Wasser | 73,85 | Gew.-% |
| Formalin (37 %ig) | 0,15 | Gew.-% |

### Beispiel 9: O/W-Emulsion

| | | |
|---|---|---|
| Eumulgin® VL 75 | 4,5 | Gew.-% |
| **Oligo-α-Olefin** | 16 | Gew.-% |
| Carbopol® | 0,3 | Gew.-% |
| KOH (20 %ig) | 0,7 | Gew.-% |
| Glycerin | 3 | Gew.-% |
| Wasser | 75,35 | Gew.% |
| Formalin (37 %ig) | 0,15 | Gew.-% |

### Beispiel 10: W/O-Emulsion

| | | |
|---|---|---|
| Dehymuls® PGPH | 5 | Gew.-% |
| Oligo-α-Olefin | 20 | Gew.-% |
| Glycerin | 5 | Gew.-% |
| Mg-Sulfat · 7 H₂O | 1 | Gew.-% |
| Wasser | 68,85 | Gew.-% |
| Formalin (37 %ig) | 0,15 | Gew.-% |

Im Weiteren sind Beispielrezepturen aufgeführt, welche die vielfältigen Einsatzmöglichkeiten der erfindungsgemäßen kosmetischen Zusammensetzungen demonstrieren. Mengenangaben beziehen sich auf Gew.-% der handelsüblichen Substanzen in der Gesamtzusammensetzung.

**Tabelle 1:O/W-Sonnenschutzemulsionen**

| **Komponente** | **1** | **2** | **3** | **4** | **5** | **6** | **7** | **8** | **9** | **10** | **11** |
|---|---|---|---|---|---|---|---|---|---|---|---|
| **L = Lotion; C=Creme;** | **L** | **C** | **S** | **L** | **C** | **L** | **L** | **C** | **L** | **C** | **L** |
| Eumulgin^{®} VL 75 | | | | | | 4 | 4 | 2 | | | |
| Eumulgin^{®} B2 | 2 | | | | | | | | | | |
| Tween^{®} 60 | | | | 1 | | | | | | | |
| Myrj^{®} 51 | | 3 | | 2 | | | | | | | |
| Cutina^{®} E 24 | 1 | | | 1 | | | | | | | |
| Hostaphat^{®} KL 340 N | | | | | | | | | 2 | | |
| Lanette^{®} E | | | 0.5 | | | | | 0.5 | | | |
| Amphisol^{®} K | | | 1 | | | 1 | | 0.5 | | 1 | |
| Natriumstearat | | | | | | | 1 | | | | 2 |
| Emulgade^{®} PL 68/50 | | | 1 | | 5 | | | | | 4 | |
| Tego^{®} Care 450 | | | | | | | | | | 3 | |
| Cutina^{®} MD | 2 | | | 6 | | | 4 | | | 6 | |
| Lanette^{®} 14 | 1 | | | 1 | | | | 2 | | | 4 |
| Lanette^{®}O | 1 | 6 | | | 5 | 2 | | 2 | | | |
| Antaron V 216 | | | 1 | | 2 | 2 | | | | 1 | |
| Emery^{®} 1780 | | | | | 0.5 | 0.5 | | | | | |
| Lanolin, wasserfrei USP | | | | | | | 5 | | | | |
| Oligo-α-Olefin (Bsp.1) | 2 | 2 | 4 | 1 | 2 | 2 | 2 | 1 | 2 | 2 | 1 |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 345 | 4 | | 1 | | 5 | | | 2 | | | 2 |
| Dow Corning DC^{®} 2502 | | 1 | | | 2 | | | | | | |
| Squatol^{®} S | | | | | | | 4 | | | | |
| Silikonöl Wacker AK^{®} 350 | | 2 | | | | | | | | | |
| Cetiol^{®} 868 | | | | | 2 | | 4 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | | | | | | | | |
| Eutanol^{®} G 16 | | | | | | | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | | | | | | | | | | | |
| Neo Heliopan^{®} Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | | 2.5 | | | 1 | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | | | 0.75 | | | | | 1 | 1 | | |
| Keltrol^{®} T | | | 0.25 | | | | | 0.5 | 0.5 | | |
| Carbopol^{®} 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5. | 5 | 5 | | 3 | 3 | 2 | | 4 | | 3 |
| Konservierungsmittel, NaOH | q.s. | | | | | | | | | | |
| Wasser | ad 100 | | | | | | | | | | |

**Tabelle 2: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **L=Lotion, C=Creme** | **L** | **L** | **L** | **C** | **L** | **C** | **S** | **C** | **C** | **L** | **L** |
| Eumulgin^{®} VL 75 | 4 | 3 | 4.5 | | 3 | | | | 4 | | |
| Eumulgin^{®} B2 | | | | | | | | | | 1 | |
| Tween^{®} 60 | | | | | | | | | | 1 | |
| Myrj^{®} 51 | | | | | | | | | | | |
| Cutina^{®} E 24 | | | | 2 | | | | | | | |
| Hostaphat^{®} KL 340 N | | | | | | | | | | 0.5 | |
| Lanette^{®} E | 0.5 | | 0.5 | 0.5 | | | 0.1 | | 0.5 | | |
| Amphisol^{®} K | 0.5 | | | | | 1 | 1 | 1 | | | |
| Natriumstearat | | | | | 1 | | | | | | |
| Emulgade^{®} PL 68/50 | | 6 | | | | 4.5 | 1 | 5 | | | |
| Tego^{®} Care 450 | 1 | | | | | | | | 4 | | |
| Cutina^{®} MD | 1 | | | 8 | 6 | 1 | | | | 4 | 1 |
| Lanette^{®} 14 | | 2 | | | | | | 2 | | 1 | |
| Lanette^{®} O | | | | 2 | | | | | 1 | 1 | |
| Antaron V 220 | 1 | | | 2 | | | 0.5 | | | 2 | 0.5 |
| Ollgo-α-Olefin (Bsp. 1) | 4 | 2 | 4 | 6 | 10 | 4 | 2 | 8 | 2 | 1 | 3 |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 345 | | 2 | | | 1 | | | | | | |
| Dow Corning DC^{®} 2502 | | 1 | | | 1 | | | | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineralöl | | | | 10 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 10 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Neo Heliopan AP (Na-Salz) | | 2 | | 2 | | | 2 | | | | 1 |
| Eusolex^{®} OCR | 6 | | 9 | | 5 | 7 | 9 | | 4 | | 7 |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} T 150 | 1 | | | | | | | | 1.3 | 1 | 1 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T-2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Pemulen^{®} TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ Na-OH | ad 100/ q.s./ q.s | | | | | | | | | | |

**Tabelle 3: W/O-Sonnenschutzemulsionen**

| **Komponente** | **23** | **24** | **25** | **26** | **27** | **28** | **29** | **30** | **31** | **32** | **33** |
|---|---|---|---|---|---|---|---|---|---|---|---|
| **L=Lotion; C=Creme** | **C** | **L** | **C** | **L** | **C** | **L** | **L** | **L** | **L** | **C** | **C** |
| Dehymuls^{®} PGPH | 4 | 2 | 1 | 3 | 3 | 1 | 1 | 2 | 2 | 4 | 1 |
| Monomuls^{®} 90-018 | | | 2 | | | | | | | | |
| Lameform^{®} TGI | 2 | | 4 | | 3 | | | | | 1 | 3 |
| Abil^{®} EM 90 | | | | | | | 4 | | | | |
| Glucate^{®} DO | | | | | | | | | | | 3 |
| Isolan^{®} PDI | | | | | | 4 | | 2 | | | |
| Arlacel^{®} 83 | | | | 2 | | | | | | | |
| Elfacos^{®} ST9 | | | | | | | | | 2 | | |
| Elfacos^{®} ST37 | | | | | | | | | | | |
| Arlacel^{®} P 135 | | 2 | | | | | | | | | |
| Dehymuls^{®} HRE 7 | | | | | | | | | | | |
| Zinkstearat | 1 | | | 1 | 1 | | | 1 | | 1 | |
| Microkristallines Wachs | | | 5 | | | 2 | | | | | 5 |
| Bienenwachs | 1 | | | 1 | | | | 5 | | 7 | |
| Tego^{®} Care CG | | | | | 1 | | | | | | 5 |
| Prisorine^{®} 3505 | 1 | | 1 | 1 | | 1 | 1 | | | | 1 |
| Emery^{®} 1780 | | | 5 | | | | | | | 4 | |
| Wollwachsalkohol, wasserfrei, USP | | | | | | | | | | | 1 |
| Antaron V 216 | 2 | | | | | | | | | | |
| Oligo-α-Olefin (Bsp. 1) | 3 | 4 | 2 | 1 | 10 | 2 | 2 | 6 | 3 | 12 | 1 |
| Myritol^{®} PC | | | | | 3 | | | 4 | | | |
| Myritol^{®} 331 | 10 | | | | 3 | 6 | | | | | 8 |
| Finsolv^{®} TN | | | | 5 | | | 5 | | | | |
| Cetiol^{®} CC | 12 | 22 | | | | 2 | | | 2 | | 5 |
| Cetiol^{®} OE | | | | | 4 | | 5 | | 4 | 2 | |
| Dow Corning DC^{®} 345 | | | | | | | 2 | | | | |
| Dow Corning DC^{®} 2502 | | | 1 | | 2 | | | | | | |
| Prisorine^{®} 3758 | | | | | | | | | | 2 | |
| Silikonöl Wacker AK^{®} 350 | | | | 4 | | | | 3 | | | |
| Cetiol^{®} 868 | | | | | | | | | | 2 | |
| Eutanol^{®} G 16 | | 3 | | | | | | | | | |
| Eutanol^{®} G 16S | | | | | | | | | | | |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G | | | 3 | | | | | 8 | | | |
| Cetiol^{®} PGL | | 11 | | | | 4 | | | 9 | | |
| Mandelöl | | | | | 1 | | 5 | | | | |
| Photonyl^{®} LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan^{®} Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan^{®} 303 | | | | | 4 | | | | | 6 | |
| Neo Heliopan^{®} BB | | 4 | 2 | | | | 2 | | | | |
| Neo Heliopan^{®} MBC | | | | | | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | | 7.5 | 7.5 | | 5 | | 7.5 |
| Uvinul^{®} T 150 | | | | | 2.5 | | | 1 | | 2 | |
| Parsol^{®} 1789 | | 2 | | | | | | 1 | | 2 | |
| Zinkoxid NDM | | | | | | 6 | | | | | |
| Eusolex^{®} T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 4: W/O-Sonnenschutzemulsionen**

| **Komponente** | **34** | **35** | **36** | **37** | **38** | **39** | **40** | **41** | **42** | **43** | **44** |
|---|---|---|---|---|---|---|---|---|---|---|---|
| **L=Lotion; C=Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 2 | 4 | 0.5 | 1 | 4 |
| Monomuls^{®} 90-018 | | 1 | | | | | | | | | |
| Lameform^{®} TGI | | | | | 4 | | | 1 | | 3 | 1 |
| Abil^{®} EM 90 | | | | 1 | | | | | | 2 | |
| Glucate^{®} DO | | | | 3 | | | | | 2 | | |
| Isolan^{®} PDI | | 3 | | | | | 4 | | | | |
| Arlacel^{®} 83 | | | | | | 3 | | | | | |
| Elfacos^{®} ST9 | | | | | | | | | | | 2 |
| Elfacos^{®} ST37 | 2 | | | | | | | | | | |
| Arlacel^{®} P 135 | | | | | | 3 | | | | | |
| Dehymuls^{®} HRE 7 | | | | | | | | | 4 | | |
| Zinkstearat | | 2 | 2 | 1 | 1 | | | 1 | 1 | | |
| Mikrokristattines Wachs | | | | | 4 | | 1 | | | 4 | |
| Bienenwachs | | 4 | | 2 | | | 1 | | 2 | | 1 |
| Tego^{®} Care CG | | | | | | | | | | | |
| Isostearinsäure | 1 | 1 | | | | | 1 | 1 | | 1 | 1 |
| Emery^{®} 1780 | | 7 | 3 | | | | | | | | |
| Wollwachsalkohol, wasserfrei, USP | | | | | | | | | | | |
| Antaron^{®} V 220 | | 0.5 | 2 | 1 | 1 | 1 | | | | | |
| Oligo-α-Olefin (Bsp. 1) | 2 | 4 | 3 | 3 | 2 | 2 | 1 | 3 | 3 | 1 | 4 |
| Myritol^{®} PC | | | | | | | | | | | |
| Myritol^{®} 331 | 4 | 2 | 3 | | 5 | | | 8 | 5 | 4 | |
| Finsolv^{®} TN | | 5 | 5 | | | 7 | | | | | |
| Cetiol^{®} CC | 3 | 1 | | | | | 3 | 16 | | | 12 |
| Cetiol^{®} OE | | 3 | | 2 | | | 3 | | | | |
| Dow Corning DC^{®} 345 | | 4 | | 2 | | | | | | | |
| Dow Corning DC^{®} 2502 | | | | 1 | | | | | | | |
| Prisorine^{®} 3578 | | 1 | | | | | | | | | |
| Silikonöl Wacker AK^{®} 350 | | | | 1 | | | | | | | |
| Cetiol^{®} 868 | | | | | | | | | | | |
| Eutanol^{®} G 16 | | | | | | | | | | | 3 |
| Eutanol^{®} G 16S | | | | | | | | | | | 7 |
| Cetiol^{®} J 600 | | | | 3 | | | | | | | |
| Ceraphyl^{®} 45 | | | | 1 | | | | | 5 | 4 | |
| Mineralöl | | | | | | | 9 | | | | |
| Cetiol^{®} B | | | | | 3 | 3 | | 2 | 2 | | |
| Eutanol^{®} G | | | | 2 | | | | | 5 | | |
| Cetiol^{®} PGL | | | | | | | | 2 | | | |
| Mandelöl | | | 2 | | | | | | | | |
| Photonyl^{®} LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan^{®} Hydro (Na-Salz) | | 4 | | | | | | 4 | | | |
| Neo Heliopan AP (Na-Salz) | 2 | | | 1 | 2 | | 1 | | | | |
| Neo Heliopan^{®} 303 | 6 | 2 | | | | | | | 6 | | |
| Neo Heliopan^{®} BB | | 2 | | 2 | | 2 | | | | | |
| Neo Heliopan^{®} MBC | 2 | | | | 3 | | 4 | | 2 | | |
| Neo Heliopan^{®} OS | | | | | 10 | | 8 | | | | |
| Neo Heliopan^{®} E 1000 | | | 5 | 6 | | | | | | 5 | |
| Neo Heliopan^{®} AV | | 5 | 5 | | | 7.5 | | | | 5 | |
| Uvinul^{®} T 150 | 1 | | | 2 | 2 | | | | 3 | 2 | |
| Parsol^{®} 1789 | | 1 | 1 | | | | 1 | | 0.5 | | |
| Z-Cote^{®} HP 1 | 4 | 10 | | | | | | 5 | | | 5 |
| Titandioxid T 805 | | | | 2 | | 3 | | 7 | | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

**Tabelle 5: W/O-Pflegeemulsionen**

| **Komponente** | **45** | **46** | **47** | **48** | **49** | **50** | **51** | **52** | **53** | **54** | **55** |
|---|---|---|---|---|---|---|---|---|---|---|---|
| **L=Lotion. C=Creme** | **C** | **L** | **C** | **L** | **C** | **L** | **L** | **L** | **C** | **C** | **C** |
| Dehymuls^{®} PGPH | 1 | 3 | 1 | 2 | 3 | 1 | 1 | 2 | 1 | 1 | 1 |
| Monomuls^{®} 90-018 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Mikrokristallines Wachs | | | 5 | | | 2 | | | | | 5 |
| Bienenwachs | 4 | | | 1 | | | | 1 | 4 | 7 | |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Emery^{®} 1780 | 1 | | | | | | | | | | 1 |
| Lanolin; anhydrous USP | | | 5 | | | | | | | 4 | |
| **Oligo-α-Oleftn (Bsp.1)** | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Corning DC^{®} 345 | | | | | 1 | | 2 | | | | |
| Dow Corning DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol/Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 6: W/O-Pflegeemulsionen**

| **Komponente** | **56** | **57** | **58** | **59** | **60** | **61** | **62** | **63** | **64** | **65** | **66** |
|---|---|---|---|---|---|---|---|---|---|---|---|
| **L = Lotion. C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 3 | 4 | 1 | 1 | 1 |
| Monomuls^{®} 90-018 | | 1 | | 1 | | | | | | | |
| Lameform^{®} TGI | | | | | 4 | | | 1 | | 3 | |
| Abil^{®} EM 90 | | | | 3 | | | | | | 2 | |
| Isolan^{®} PDI | | 3 | | | | | | | | | 4 |
| Glucate^{®} DO | 1 | | | | | | | | | | |
| Arlacel^{®} 83 | | | | | | 3 | | | | | |
| Dehymuls^{®} FCE | | | | | 4 | | 1 | | | | |
| Dehymuls^{®} HRE 7 | | | | | | | | | 7 | | |
| Zinkstearat | | 2 | 2 | 1 | 1 | 1 | | 1 | 1 | | 1 |
| Mikrokristallines Wachs | | | | | 4 | | 1 | | | 4 | |
| Bienenwachs | | 4 | | 2 | | 2 | 1 | 1 | 2 | | 5 |
| Tego^{®} Care CG | | | | | | | | | | | |
| Prisorine^{®} 3505 | 1 | 1 | | | | | 1 | 1 | | 1 | 1 |
| Dry Flo^{®} Plus | 1 | | | | | | | | | | |
| SFE^{®} 839 | | 5 | | | | 4 | | | | | |
| Emery^{®} 1780 | | | | | | | | | | | |
| Lanolin anhydrous USP | | 7 | 3 | | | | | | | | |
| Oligo-α-Olefin (Bsp. 1) | 3 | 4 | 4 | 8 | 10 | 2 | 8 | 6 | 3 | 12 | 7 |
| Cegesoft^{®} C 17 | | 2 | | | | | | | | | |
| Myritol^{®} PC | | | | 8 | | | | | | | |
| Myritol^{®} 331 | 4 | | 3 | | 5 | 3 | | | 5 | 4 | |
| Finsolv^{®} TN | | | 5 | | | 7 | | | | | |
| Cetiol^{®} A | | | | | | | | 6 | | | |
| Cetiol^{®} CC | 3 | | | 6 | | 3 | 3 | | | 8 | |
| Cetiol^{®} SN | | | | | 5 | | | | | | |
| Cetiol^{®} OE | | 3 | | 2 | | | 3 | | | | 8 |
| Dow Corning ^{®} DC 345 | | 4 | | 2 | | 2 | | | | | |
| Dow Corning^{®} DC 2502 | | | | 1 | | | | | | | |
| Prisorine^{®} 3758 | | | | | | 1 | | | | | |
| Silikonöl Wacker AK^{®} 350 | | | | 1 | | 1 | | 4 | | | |
| Cetiol^{®} 868 | | | | | | | | | | | 10 |
| Cetiol^{®} J 600 | 4 | | | 3 | | | | | | | |
| Ceraphyl^{®} 45 | | | | 1 | | | | | 5 | 4 | |
| Mineralöl | | | | | | | 9 | | | | |
| Cetiol^{®} B | | | | | 3 | 3 | | 2 | 2 | | |
| Eutanol^{®} G 16 | 1 | | | | | | | | | | |
| Eutanol^{®} G | | | | 2 | | | | | 5 | | |
| Cetiol^{®} PGL | | | 10 | | | | | 6 | | | 3 |
| Mandelöl | | | 2 | | 5 | | 2 | | | | |
| Photonyl^{®} LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone^{®} 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

**Tabelle 7: O/W-Pflegeemulsionen**

| **Komponente** | **67** | **68** | **69** | **70** | **71** | **72** | **73** | **74** | **75** | **76** | **77** |
|---|---|---|---|---|---|---|---|---|---|---|---|
| **L=Lotion, C=Creme** | **C** | **C** | **C** | **L** | **C** | **L** | **L** | **C** | **L** | **C** | **C** |
| Eumulgin^{®} VL 75 | | | | | | 4 | | | | | |
| Dehymuls^{®} PGPH | | 2 | | | | | | | | | |
| Generol^{®} R | | | 1 | | | | | | | | |
| Eumulgin^{®} B2 | | | 0.8 | | | | | | | | |
| Tween^{®} 60 | | | | 1 | | | | | | | |
| Cutina^{®} E24 | | | 0.6 | 2 | | | | | | | |
| Hostaphat^{®} KL 340 N | | | | | | | | | 2 | | |
| Lanette^{®} E | | | | | | | | 1 | | | |
| Amphisol^{®} K | | 0.5 | | | | 1 | | | | 1 | 0.5 |
| Natriumstearat | | | | | 0.5 | | | | | | |
| Emulgade^{®} PL 68/50 | | 2.5 | | | | | | | | 4 | |
| Tego^{®} Care CG | | | | | | | | | | | 2 |
| Tego^{®} Care 450 | | | | | | | | 5 | | | |
| Cutina^{®} MD | | 1 | | 6 | 5 | | 4 | | | 6 | |
| Lanette^{®} 14 | | | | 1 | | | | 2 | | | 4 |
| Lanette^{®} O | 4.5 | | 4 | | 1 | 2 | | | | | 2 |
| Novata^{®} AB | | 1 | | | | | | | | | 1 |
| Emery^{®} 1780 | | | | | 0.5 | 0.5 | | | | | |
| Lanolin, wasserfrei, USP | | | | | | | 5 | | | | |
| Cetiol^{®} SB 45 | | | 1.5 | | | | 2 | | | | |
| Oligo-α-Olefin (Bsp. 1) | 3 | 4 | 2 | 1 | 10 | 2 | 2 | 6 | 3 | 12 | 1 |
| Cegesoft^{®} C 17 | | | | | | | | | | | |
| Myritol^{®} PC | | | | | 5 | | | | | | |
| Myritol^{®} 331 | 2 | 5 | 5 | | | 6 | | 12 | | | |
| Finsolv^{®} TN | | | 2 | | | 2 | | | 8 | | |
| Cetiol^{®} CC | 4 | 6 | | | | 4 | 4 | | | | 5 |
| Cetiol^{®} OE | | | | | | | | | 4 | 3 | |
| Dow Corning DC^{®} 345 | | | 2 | | 5 | 1 | | | | | |
| Dow Corning DC^{®} 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineralöl | | | | 9 | | | | | | | |
| Cetiol^{®} SN | | | 5 | | | | | | | | |
| Cetiol^{®} B | | | | | | | | | | 2 | |
| Eutanol^{®} G | | 2 | | 3 | | | | | | | |
| Cetiol^{®} PGL | | | | | | | | | 5 | 5 | |
| Dry Flo^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | . | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Pemulen^{®} TR 2 | 0.3 | | | | | | | 0.3 | | | |
| Carbopol^{®} Ultrez 10 | | 0.3 | 0.3 | 0.2 | 0.2 | 0.2 | | | 0.1 | 0.3 | 0.2 |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, | ad 100, q.s., | | | | | | | | | | |
| NaOH | pH 6,5 - 7,5 | | | | | | | | | | |

**Tabelle 8: /W-Pflegeemulsionen**

| **Komponent** | **78** | **79** | **80** | **81** | **82** | **83** | **84** | **85** | **86** | **87** | **88** |
|---|---|---|---|---|---|---|---|---|---|---|---|
| **L=Lotion, C = Creme** | **C** | **C** | **L** | **C** | **L** | **C** | **L** | **L** | **L** | **L** | **C** |
| Eumulgin^{®} VL 75 | 4 | 3 | | | | | 1 | | | | 2 |
| Generol^{®} R | | | | | | 2 | | | | | |
| Eumulgin^{®} B2 | | | | | | 2 | | | | 1 | |
| Tween^{®} 60 | | | | | | | | | | 1 | |
| Cutina^{®} E 24 | | | | 2 | | | | | | | |
| Hostaphat^{®} KL 340 N | | | | | | | | | | | |
| Lanette^{®} E | 0.5 | | | | | | | | | | 1 |
| Amphisol^{®} K | 0.5 | 1 | | | | | | 1 | 1 | | |
| Natriumstearat | | | | | 1 | | | | | | |
| Emulgade^{®} PL 68/50 | | 6 | | | | | | 5 | | | 4 |
| Tego^{®} Care CG | | | | | | | | | | | |
| Tego^{®} Care 450 | | | | | | | | | 4 | | |
| Cutina^{®} MD | 3 | | 3 | 8 | 6 | 8 | | | | 4 | |
| Lanette^{®} 14 | | 2 | | | | | | 2 | | 1 | |
| Lanette^{®} O | 2 | | | 2 | | 3 | 1 | | 1 | 1 | 6 |
| Novata^{®} AB | | | | | | | | | | | |
| Emery^{®} 1780 | | | | | | | | | | | |
| Lanolin, wasserfrei, USP | | | | | | 4 | | | | | |
| Cetiol^{®} SB 45 | | | | | | | 2 | | | | |
| Ongo-α-Olefln (Bsp. 1) | 3 | 4 | 2 | 1 | 10 | 2 | 2 | 6 | 3 | 12 | 1 |
| Cegesoft^{®} C 17 | 4 | | | | | | | | | | |
| Myritol^{®} PC | 6 | | | | | 5 | | | 5 | | |
| Myritol^{®} 331 | 5 | | 5 | | | | 7 | | | 10 | 3 |
| Finsolv^{®} TN | | 5 | | | 5 | | | 3 | 3 | | 1 |
| Cetiol^{®} CC | | | | | | | | | | | 2 |
| Cetiol^{®} OE | | | | | 2 | | 2 | | 5 | | |
| Dow Corning DC^{®} 345 | | 2 | | | 1 | | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | | | | | | | | |
| Ceraphyl^{®} 45 | | | | | | | 3 | | | | |
| Cetiol^{®} SN | | | | | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Carbopol^{®} ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, | ad 100, q.s. | | | | | | | | | | |
| NaOH | (pH 6,5 - 7,5) | | | | | | | | | | |

**Tabelle 9: Sprayformullerungen**

| **Komponente** | **89** | **90** | **91** | **92** | **93** | **94** | **95** | **96** | **97** | **98** | **99** |
|---|---|---|---|---|---|---|---|---|---|---|---|
| **S= Körperspray, S*=Sonnenschutzspray.** | **S** | **S** | **S** | **S** | **S** | **S*** | **S*** | **S*** | **S*** | **S*** | **S*** |
| Emulgade^{®} SE-PF | 8,9 | | 7,5 | 7.5 | 4,3 | 9.8 | 8.2 | 9.9 | | | |
| Eumulgin^{®} B2 | 3,1 | | 3 | | | | | 4.2 | | | |
| Eumulgin^{®} B3 | | | | | | 4.2 | 3.3 | | | | |
| Eumulgin^{®} HRE 40 | | | | | 4,7 | | | | | | |
| Cutina^{®} E 24 | | 5,9 | | 4 | | | | | | | |
| Amphisol^{®} K | | | | | | | | | 1 | 1 | 1 |
| Eumulgin^{®} VL 75 | | | | | | | | | | | 2 |
| Emulgade^{®} PL 68/50 | | 0.5 | | | | | | | 2.5 | 1 | |
| Cutina^{®} MD | | 3,1 | | | | | | | | | |
| Antaron^{®} V 220 | | | | | | 1 | 1 | 1 | | 1 | 1 |
| Oligo-α-Olefin (Bsp. 1) | 11 | 5 | 7 | 7 | 7 | 5 | 4 | 5 | 5 | 4 | 6 |
| Myritol^{®} PC | | | | | | | | | | | |
| Myritol^{®} 331 | | | 3 | 4 | 3 | 3 | 3 | 3 | | | |
| Finsolv^{®} TN | | 4 | | | | | | 8 | | | |
| Cetiol^{®} CC | 6 | | | 5 | 5 | 2 | 2 | 4 | | | |
| Cetiol^{®} OE | | 5 | 5 | | | 2 | | | | | |
| Dow Corning DC^{®} 345 | | 4 | 4 | 5 | | | | | | | |
| Cetiol^{®} 868 | 3 | | | | | | | | | | |
| Cetiol^{®} J 600 | | | | 2 | 2 | | | | | | |
| Mineralöl | | | 2 | | | | | | | | |
| Cetiol^{®} B | | | | | | | 2 | | | | |
| Eutanol^{®} G | 2 | | | | 1 | | | | | | |
| Photonyl^{®} LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | 2 | | | | 3 | |
| Neo Heliopan AP (Na-Salz) | | | | | | 2 | 2 | 2 | | | 1 |
| Eusolex^{®} OCR | | | | | | | 2 | | | | 3 |
| Neo Heliopan^{®} BB | | | | | | | | | | 1 | |
| Neo Heliopan^{®} MBC | | | | | | 2 | 2 | 2 | | 1 | 1 |
| Neo Heliopan^{®} OS | | | | | | 5 | | | | | |
| Neo Heliopan^{®} AV | | | | | | 6 | 6 | 2 | | 7.5 | 2 |
| Uvinul^{®} T 150 | | | | | | 1 | 1 | 1 | | 1 | |
| Parsol^{®} 1789 | | | | | | 1 | | 1 | | 1 | |
| Z-Cote^{®} HP 1 | | | | | | | | | | 2 | 2 |
| Eusolex^{®} T 2000 | | | | | | | | | | 2 | 2 |
| Veegum^{®} Ultra | | | | | | | | | | | 1.5 |
| Laponite^{®}XLG | | | | | | | | | | 1.5 | |
| Keltrol^{®} T | | | | | | | | | | | 0.5 |
| Pemulen^{®} TR 2 | | | | | | | | | 0.2 | | |
| Insect Repellent^{®} 3535 | 1 | | | | | | | | | | |
| N,N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |
| Wasser/ Konservierungsmittel/ NaOH | ad 1001 q.s./ q.s | | | | | | | | | | |

**Tabelle 10: AP/Deo-Formulierungen**

| **Komponente** | **100** | **101** | **102** | **103** | **104** | **105** | **106** | **107** | **108** | **109** | **110** |
|---|---|---|---|---|---|---|---|---|---|---|---|
| S=Stiff; SO = Soft solid; RA = Roll-on wasserfrei; A = Aerosol; RH = Roll-on wassing;C = Creme | **S** | **S** | **SO** | **SO** | **RA** | **RA** | **A** | **A** | **RH** | **RH** | **C** |
| Lanette^{®} 18 | 14.7 | 14.7 | | | | | | | | | |
| Cutina^{®} HR | 3.7 | 3.7 | 6.5 | 6.5 | | | | | | | |
| Emulgade^{®} NLB | | | | | | | | | 5.0 | | |
| Eumulgin^{®} S2 | | | | | | | | | | 3.0 | |
| Eumulgin^{®} S21 | | | | | | | | | | 2.5 | |
| Eumugade^{®} SE | | | | | | | | | | | 6.0 |
| Lanette^{®} 22 | | | | | | | | | | | 2.0 |
| Novata^{®} B | | | 10.0 | 10.0 | | | | | | | |
| Dow Coming^{®} DC 245 | | 15.0 | 14.0 | | 20.0 | 10.0 | | | | | |
| Cetiol^{®} OE | | 9.0 | 9.0 | 5.0 | 5.0 | 5.0 | | | | | 3.0 |
| Cetiol^{®} S | | 14.7 | 14.0 | 10.0 | 5.0 | | | | | | |
| Finsolv^{®} TN | | | | | | 10.0 | | | | | |
| Cetiol^{®} CC | | | | | | | 3.0 | | 4.0 | 2.0 | |
| Eutanol^{®} G16 | | 5.0 | | | | 10.0 | | | | | |
| Oligo-α-Olefin (Bsp.1) | 58.7 | 15.0 | 15.0 | 37.0 | 40.0 | 35.0 | 10.0 | 13.0 | 2.0 | 4.0 | 6.0 |
| Dow Corning^{®} DC 200 | | | | | | | | | 1.0 | 1.0 | 1.0 |
| Miglyol^{®} Gel B | | | 0,5 | 0.5 | 1.5 | 1.5 | 4.0 | 4.0 | | | |
| Bentone^{®} 38 | | | 1.0 | 1.0 | 4.5 | 4.5 | | | | | |
| Talkum | | | 5.0 | 5.0 | | | | | | | |
| Rezal^{®} 36 GP | 22.9 | | 25.0 | | 24.0 | | | | | | |
| Rezal^{®} 36 GC | | | | | | | | | | | 30.0 |
| Locron^{®} P | | 22.9 | | 25.0 | | 24.0 | 6.0 | 8.0 | | | |
| Locron^{®} L | | | | | | | | | 40.0 | 40.0 | |
| Hydagen^{®} CAT | | | | | | | 2.0 | | | | |
| Hydagen^{®} DCMF | | | | | | | | | | | 0.05 |
| Treibgas (Butan/Propan) | | | | | | | 75.0 | 75.0 | | | |
| Wasser | | | | | | | | | 48.0 | 47.5 | 51.95 |
| Konservierungsmittel | qs | qs | qs | qs | qs | qs | qs | qs | qs | qs | qs |

### ANHANG

| | |
|---|---|
| 1) Abil^{®} EM 90 | |
| INCI: Cetyl Dimethicone Copolyol | 15) cedol^{®} A |
| Hersteller: Tego Cosmetics (Goldschmidt) | INCI: Hexyl Laurate |
| | Hersteller: Cognis Deutschland GmbH |
| 2) Amphlsol^{®} K | |
| INCI: Potassium Cetyl Phosphate | 16) Cetiol^{®} B |
| Hersteller: Hoffmann La Roche | INCI: Butyl Adipate |
| | Hersteller: Cognis Deutschland GmbH (Henkel) |
| 3) Antaron^{®} V 220 | |
| INCI: PVP/Eicosene Copolymer | 17) Cetiol^{®}J 600 |
| Hersteller: GAF General Aniline Firm Corp. (IPS-Global) | INCI: Oleyl Erucate |
| | Hersteller: Cognis Deutschland GmbH |
| 4) Antaron^{®} V 216 | |
| INCI: PVP/Hexadecene Copolymer | 18) Cetiol^{®} OE |
| Hersteller: GAF General Aniline Firm Corp. (IPS-Global) | INCI: Dicaprylyl Ether |
| | Hersteller: Cognis Deutschland GmbH |
| 5) Arlacel^{®} 83 | |
| INCI: Sorbitan Sesquioteate | 19) Cetiol^{®} PGL |
| Hersteller: Uniqema (ICI Surfacants) | INCI: Hexyldecanol, Hexyldecyl Laurate |
| | Hersteller: Cognis Deutschland GmbH |
| 6) Ariacel^{®} P 135 | |
| INCI: PEG-30 Dipolyhydroxystearate | 20) Cetiol^{®} CC |
| Hersteller: Uniqema (ICI Surfacants) | INCI: Dicaprylyl Carbonate |
| | Hersteller: Cognis Deutschland GmbH |
| 7) Bentone^{®} 38 | |
| INCI: Quatemlum-18 Hectorite | 21) Cetiol^{®} SB 45 |
| Hersteller: Rheox (Elementis Specialties) | INCI: Shea Butter Butyrospermum Parkii (Unne) |
| | Hersteller: Cognis Deutschland GmbH |
| 8) Carbopol^{®} 980 | |
| INCI: Carbomer | 22) Cetiol^{®} SN |
| Hersteller: Goodrich | INCI: Cetearyl Isononanoate |
| | Hersteller: Cognis Deutschland GmbH (Henkel) |
| 9) Carbopol^{®} 2984 | |
| INCI: Carbomer | 23) Cutina^{®} E 24 |
| Hersteller: Goodrich | INCI: PEG-20 Glyceryl Stearate |
| | Hersteller: Cognis Deutschland GmbH |
| 10) Carbopol^{®} ETD 2001 | |
| INCI: Carbomer | 24) Cutina^{®} MD |
| Hersteller: BF Goodrich | INCI: Glyceryl Stearate |
| | Hersteller: Cognis Deutschland GmbH |
| 11) Carbopol^{®} Ultrez 10 | |
| INCI: Carbomer | 25) Dehymuls^{®} FCE |
| Hersteller Goodrich | INCI: Dicocoyl Pentaerythrityl Distearyl Citrate |
| | Hersteller: Cognis Deutschland GmbH |
| 12)' Cegesoft^{®} C 17 | |
| INCI: Myristyl Lactate | 26) Dehymuls^{®} HRE 7 |
| Hersteller: Cognis Deutschland GmbH, Grünau | INCI: PEG-7 Hydrogenated Castor Oil |
| | Hersteller: Cognis Deutschland GmbH |
| 13) Ceraphyl^{®} 45 | |
| INCI: Diethylhexyl Malate | 27) Dehymuls^{®} PGPH |
| Hersteller: International Specialty Products | INCI: Polyglyceryl-2 Dipolyhydroxystearate |
| | Hersteller Cognis Deutschland GmbH |
| 14) Cetiol^{®} 868 | |
| INCI: Ethylhexyl Stearate | 28) Dow Corning^{®} 345 Fluid |
| Hersteller: Cognis Deutschland GmbH | INCI: Cyclomethicone |
| | Hersteller: Dow Corning |
| 29) Dow Coming^{®} 245 Fluid | 43) Eutanol^{®}G 16 S |
| INCI: Cyclopentasiloxane Cyclomethicone | INCI: Hexyldecyl Stearate |
| Hersteller Dow Corning | Hersteller. Cognis Deutschland GmbH |
| | |
| 30) Dow Corning^{®} 2502 | 44) Finsolv^{®} TN |
| INCI: Cetyl Dimethicone | INCI: C 12/15 Alkyl Benzoate |
| Hersteller: Dow Corning | Hersteller. Andex (Nordmann/Rassmann) |
| | |
| 31) Dry^{®}Flo Plus | 45) Generol^{®} R |
| INCI: Aluminium Starch Octenylsuccinate | INCI: Brassica Campestris (Rapseed) Sterols |
| Hersteller. National Starch | Hersteller: Cognis Deutschland GmbH |
| | |
| 32) Elfacos^{®}ST 37 | 46) Glucate^{®} DO |
| INCI: PEG-22 Dodecyl Glycol Copolymer | INCI: Methyl Glucose Dioleate |
| Hersteller: Akzo-Nobel | Hersteller. NRC Nordmann/Rassmann |
| | |
| 33) Elfacos^{®}ST 9 | 47) Hostaphat^{®} KL 340 N |
| INCI: PEG-45 Dodecyl Glycol Copolymer | INCI: Trilaureth -4 Phosphate |
| Hersteller. Akzo-Nobel | Hersteller: Clariant |
| | |
| 34) Emery^{®} 1780 | 48) Isolan^{®} PDI |
| INCI: Lanolin Alcohol | INCI: Diisostearoyl Polyglyceryl-3 Diisostearate |
| Herstellar: Cognis Corporation (Emery) | Hersteller: Goldschmidt AG |
| | |
| 35) Emulgade^{®} PL 68/50 | 49) Keltrol^{®} T |
| INCI: Cetearyl Glucoside, Ceteayl Alcohol | INCI: Xanthan Gum |
| Hersteller: Cognis Deutschland GmbH | Hersteller: CP Kelco |
| | |
| 36) Emulgade^{®} SE-PF | 50) Lameform^{®}TGl |
| INCI: Glyceryl Stearate, Cateareth-20, Ceteareth-12, | INCI: Polyglyceryl-3 Diisostearate |
| Cetearyl Alcohol, Cetyl Palmitate | Hersteller: Cognis Deutschland GmbH |
| Hersteller: Cognis Deutschland GmbH | |
| | 50) Lanette^{®} 14 |
| 37) Eumulgin^{®} B 2 | INCI: Myristyl Alcohol |
| INCI: Ceteareth- 20 | Hersteller: Cognis Deutschland GmbH |
| Hersteller: Cognis Deutschland GmbH | |
| | 51) Lanette^{®} E |
| 38) Eumulgln^{®} VL 75 | INCl: Sodium Cetearyl Sulfate |
| INCl: Lauryl Glucoside (and) Polyglyceryl-2 Dipolyhydro- | Hersteller. Cognis Deutschland GmbH |
| xystearate (and) Glycerin | |
| Hersteller Cognis Deutschland GmbH | 52) Lanette^{®} O |
| | INCl: Cetearyl Alcohol |
| 39) Eusolex^{®} OCR | Hersteller. Cognis Deutschland GmbH |
| INCl: Octocrylene | |
| Hersteller: Merck | 53) Monomuls^{®} 90-0-18 |
| | INCl: Glyceryl Oleate |
| 40) Eusolex^{®} T 2000 | Hersteller: Cognis Deutschland GmbH |
| INCI: Titanium Dioxide, Alumina, Simethicone | |
| Hersteller: Rona (Merck) | 54) Myri^{®} 51 |
| | INCI: PEG-30-Sterate |
| 41) Eutanol^{®}G | Hersteller: Uniqema |
| INCI: Octyidodecanol | |
| Hersteller: Cognis Deutschland GmbH | 55) Myritol^{®} 331 |
| | INCI: Cocoglycerides |
| 42) Eutanol^{®}G 16 | Hersteller: Cognis Deutschland GmbH |
| INCI: Hexyldecanol | |
| Hersteller: Cognis Deutschland GmbH | |
| | |
| 56) Myritol^{®} PC | 69) Frisorine^{®} ISAC 3505 |
| INCI: Propylene Glycol Dicaprylate/Dicaprate | INCI: Isostearic Acid |
| Hersteller Cognis Deutschland GmbH | Hersteller: Uniqema |
| | |
| 57) Neo Heliopan^{®} 303 | 70) Prisorine^{®} 3758 |
| INCI: Octocrylene | INCI: Hydrogenated Polyisobutene |
| Hersteller: Haarmann & Reimer | Hersteller: Uniqema |
| | |
| 58) Neo Hellopan^{®} AP | 71) Ravecarb^{®} 106 |
| INCI: Disodium Phenyl Dibenzimidazole Tetrasulfonate | Polycarbonatdiol |
| Hersteller. Haarmann & Reimer | Hersteller: Enichem |
| | |
| 59) Neo Heliopan^{®} AV | |
| INCI: Ethylhexyl Methoxycinnamate | 73) SFE^{®} 839 |
| Hersteller. Haarmann & Reimer | INCI: Cyclopentasiloxane and Dimethicone/Vinyl Dl- |
| | methicone Crosspolymer |
| 60) Neo Heliopan^{®} BB | Hersteller. GE Silicones |
| INCI: Benzophenone-3 | |
| Hersteller: Haarmann & Reimer | 74) Silikonöl Wacker AK^{®} 350 |
| | INCI: Dimethicone |
| 61) Neo Heliopan^{®} E 1000 | Hersteller: Wacker |
| INCI: Isoamyl-p-Methoxyannamate | |
| Hersteller: Haarmann & Reimer | 75) Squatol^{®} S |
| | INCI: Hydrogenated Polyisobutene |
| 62) Neo Heliopan^{®} Hydro (Na-Salz) | Hersteller: LCW (7-9 rue de l'Industrie 95310 St-Ouen |
| INCI: Phenylbenzimldazole Sulfonic Acid | l'Aumone France) |
| Hersteller: Haarmann & Reimer | |
| | 76) Tego^{®} Care 450 |
| 63) Neo Heliopan^{®} MBC | INCI: Polyglyceryl-3 Methylglucose Distearate |
| INCI: 4-Methylbenzylidene Camphor | Hersteller: Tego Cosmetics (Goldschmidt) |
| Hersteller: Haarmann & Reimer | |
| | 77) Tego^{®} Care CG 90 |
| 64) Neo Heliopan^{®} OS | INCl: Cetearyl Glucoside |
| INCl: Ethylhexyl Salicylate | Hersteller: Goldschmidt |
| Hersteller: Haarmann & Reimer | |
| | 78) Tween^{®} 60 |
| 65) Novata^{®} AB | INCl: Polysorbate 60 |
| INCl: Cocoglycerides | Hersteller: Uniqema (ICI Surfactants) |
| Hersteller: Cognis Deutschland GmbH | |
| | 79) Uvinul^{®} T 150 |
| 66) Parsol^{®} 1789 | INCl: Octyl Triazone |
| INCI: Butyl Methoxydibenzoylmethane | Hersteller. BASF |
| Hersteller: Hoffmann-La Roche (Glvaudan) | |
| | 80) Veegum^{®} Ultra |
| 67) Pemulen^{®} TR-2 | INCI: Magnesium Aluminium Silicate |
| INCI: Acrylates / C10-30 Alkylacrylate Crosspolymer | Hersteller: Vanderblit |
| Hersteller: Goodrich | |
| | 81) Z-Cote^{®} HP 1 |
| 68) Photonyl^{®} LS | INCI: Zinc Oxide, Dimethicone |
| INCI: Arginine, Disodium Adenosine Triphosphate, Man- | Hersteller. BASF |
| nitol, Pyridoxine HCL, Phenylalanine, Tyrosine | |
| Hersteller: Laboratoires Seroblologiques (Cognis) | |

## Patentansprüche

1. Kosmetische Zusammensetzung enthaltend wenigstens ein verzweigtes Oligo-α-Olefin, **dadurch gekennzeichnet, dass** die Seitenketten an wenigstens einer Verzweigungsstelle Ethyl-, Propyl- oder längerkettige verzweigte Alkylreste sind, weiches **dadurch** erhältlich ist, dass
a) wenigstens ein verzweigtes α-Olefin mit 5 bis 18 Kohlenstoffatomen,
b) wenigstens ein lineares α-Olefin mit 4 bis 10 Kohlenstoffatomen,
c) ein Gemisch aus einem verzweigten α-Olefin mit 4 bis 18 Kohlenstoffatomen und einem linearen α-Olefin mit 3 bis 18 Kohlenstoffatomen oder
d) ein Gemisch aus verschiedenen, verzweigten α-Olefinen mit 4 bis 18 Kohlenstoffatomen und linearen α-Olefinen mit 3 bis 18 Kohlenstoffatomen
in Gegenwart eines Katalysators - ausgewählt aus der Gruppe der organischen Säuren, der kationischen lonenenatistauscher, Kieselgele, Schichtsilikate, anorganischen Säuren oder Lewis-Säure basierten Katalysatoren - oligomerislert wird.

2. Kosmetische Zusammensetzung nach Anspruch 1, **dadurch gekennzeichnet, dass** das verzweigte Oligo-α-Olefin insgesamt 12 bis 36, vorzugsweise 12 bis 24 und insbesondere 14 bis 24 Kohlenstoffatome aufweist.

3. Kosmetische Zusammensetzung nach Anspruch 1 oder 2, **dadurch gekennzeichnet, dass** das aus der Oligomerisierung resultierende, verzweigte Oligo-α-Olefin anschließend hydriert wird.

4. Kosmetische Zusammensetzung nach wenigstens einem der Ansprüche 1 bis 3, **dadurch gekennzeichnet, dass** ein Gemisch.(c) aus einem verzweigten α-Olefin mit 5 bis 12 Kohlenstoffatomen und einem linearen α-Olefin mit 3 bis 12 Kohlenstoffatomen in Gegenwart eines Katalysators - ausgewählt aus der Gruppe der organischen Säuren, der kationischen lorienenaustauscher, Kieselgele, Schichtsilikate, anorganischen Säuren oder Lewis-Säure basierten Katalysatoren - oligomerisiert wird.

5. Kosmetische Zusammensetzung nach wenigstens einem der Ansprüche 1 bis 4, **dadurch gekennzeichnet, dass** das lineare α-Olefin ausgewählt ist aus der Gruppe 1-Propen, 1-Buten, 2-Buten, 1-Penten und 2-Penten.

6. Kosmetische Zusammensetzung nach wenigstens einem der Ansprüche 1 bis 5, **dadurch gekennzeichnet, dass** das verzweigte α-Olefin ausgewählt ist aus der Gruppe. 2-Ethyl-1-hexen, 2-Propylhepten, 2-Methyl-1-buten, 2-Methyl-1-penten, 3-Methyl-1-penten, oder 4-Methyl-1-penten.

7. Kosmetische Zusammensetzung nach wenigstens einem der Ansprüche 1 bis 6, **dadurch gekennzeichnet, dass** ein Gemisch aus 80 % Buten und 20 % iso-Buten in Gegenwart eines Katalysators - ausgewählt aus der Gruppe der organischen Säuren, der kationischen lonenaustauscher, Kieselgele, Schichtsilikate, anorganischen Säuren oder Lewis-Säure basierten Katalysatoren - oligomerisiert wird.

8. Kosmetische Zusammensetzung nach wenigstens einem der Ansprüche 1 bis 7, **dadurch gekennzeichnet, dass** sie in Form einer W/O- oder O/W-Emuision vorliegt.

9. Kosmetische Zusammensetzung nach wenigstens einem der Ansprüche 1 bis 8, **dadurch gekennzeichnet, dass** sie 1 bis 50 Gew.-%, bevorzugt 5 bis 40 Gew.-% und insbesondere 5 bis 25 Gew.-%,Öikörper enthält.

10. Kosmetische Zusammensetzung nach einem der Ansprüche 1 bis 9, **dadurch gekennzeichnet, dass** der Anteil des wenigstens einen Oligo-α-Olefins bezogen auf die Gesamtmenge der Ölkörper 0,1 bis 100 Gew.-%, bevorzugt 1 bis 50 Gew.-%, besonders bevorzugt 1 bis 20 Gew.-% beträgt.

11. Kosmetische Zusammensetzung nach einem der Ansprüche 1 bis 10, **dadurch gekennzeichnet, dass** sie 0,1 bis 20 Gew.-% vorzugsweise 1 -15 Gew.-% und insbesondere 1 -10 Gew.-% einer oberflächenaktiven Substanz oder eines Gemisches aus oberflächenaktiven Substanzen enthält

12. Kosmetische Zusammensetzung nach einem der Ansprüche 1 bis 11, **dadurch gekennzeichnet, dass** sie zusätzlich wenigstens einen antitranspiranten und/oder Deo-Wirkstoff enthält.

13. Verwendung von Oligo-α-Olefin, weiches **dadurch** erhältlich ist, dass man
(a) wenigstens ein verzweigtes α-Olefin mit 5 bis 18 Kohlenstoffatomen,
(b) wenigstens ein lineares α-Olefin mit 4 bis 10 Kohlenstoffatomen,
(c) ein Gemisch aus einem verzweigten α-Olefin mit 4 bis 18 Kohlenstoffatomen und einem linearen α-Olefin mit 3 bis 18 Kohlenstoffatomen oder
(d) ein Gemisch aus verschiedenen, verzweigten α-Olefinen mit 4 bis 18 Kohlenstoffatomen und linearen α-Olefinen mit 3 bis 18 Kohlenstoffatomen
in Gegenwart eines Katalysators - ausgewählt aus der Gruppe der organischen Säuren, der kaüonischen lonenaustauscher, Kieselgele, Schichtsifikate, anorganischen Säuren oder Lewis-Säure basierten Katalysatoren - oligomerisiert und anschließend gegebenenfalls hydriert, als Ölkörper in kosmetischen oder pharmazeutischen Zubereitungen, insbesondere in Antitranspirant und/oder Deo-Formulierungen.

## Claims

1. Cosmetic composition containing at least one branched oligo-α-olefin, **characterized in that** the side chains at one branch point at least are ethyl, propyl or longer branched alkyl chains, the branched oligo-α-olefin being obtainable by oligomerization of
a) at least one branched α-olefin containing 5 to 18 carbon atoms,
b) at least one linear α-olefin containing 4 to 10 carbon atoms,
c) a mixture of a branched α-olefin containing 4 to 18 carbon atoms and a linear α-olefin containing 3 to 18 carbon atoms or
d) a mixture of various branched α-olefins containing 4 to 18 carbon atoms and linear α-olefins containing 3 to 18 carbon atoms
in the presence of a catalyst selected from the group consisting of organic acids, cationic ion exchangers, silica gels, layer silicates, inorganic acids or Lewis-acid-based catalysts.

2. Cosmetic composition as claimed in claim 1, **characterized in that** the branched oligo-α-olefin contains a total of 12 to 36, preferably 12 to 24 and more particularly 14 to 24 carbon atoms.

3. Cosmetic composition as claimed in claim 1 or 2, **characterized in that** the branched oligo-α-olefin resulting from the oligomerization is subsequently hydrogenated.

4. Cosmetic composition as claimed in at least one of claims 1 to 3, **characterized in that** a mixture (c) of a branched α-olefin containing 5 to 12 carbon atoms and a linear α-olefin containing 3 to 12 carbon atoms is oligomerized in the presence of a catalyst selected from the group consisting of organic acids, cationic ion exchangers, silica gels, layer silicates, inorganic acids or Lewis-acid-based catalysts.

5. Cosmetic composition as claimed in at least one of claims 1 to 4, **characterized in that** the linear α-olefin is selected from the group consisting of 1-propene, 1-butene, 2-butene, 1-pentene and 2-pentene.

6. Cosmetic composition as claimed in at least one of claims 1 to 5, **characterized in that** the branched α-olefin is selected from the group consisting of 2-ethyl-1-hexene, 2-propyl heptene, 2-methyl-1-butene, 2-methyl-1-pentene, 3-methyl-1-pentene or 4-methyl-1-pentene.

7. Cosmetic composition as claimed in at least one of claims 1 to 6, **characterized in that** a mixture of 80% butene and 20% isobutene is oligomerized in the presence of a catalyst selected from the group of organic acids, cationic ion exchangers, silica gels, layer silicates, inorganic acids or Lewis-acid-based catalysts.

8. Cosmetic composition as claimed in at least one of claims 1 to 7, **characterized in that** it is present in the form of a w/o or o/w emulsion.

9. Cosmetic composition as claimed in at least one of claims 1 to 8, **characterized in that** it contains 1 to 50% by weight, preferably 5 to 40% by weight and more particularly 5 to 25% by weight of oil components.

10. Cosmetic composition as claimed in any of claims 1 to 9, **characterized in that** the percentage content of the at least one oligo-α-olefin, based on the total quantity of oil components, is 0.1 to 100% by weight, preferably 1 to 50% by weight and more preferably 1 to 20% by weight.

11. A cosmetic composition as claimed in any of claims 1 to 10, **characterized in that** it contains 0.1 to 20% by weight, preferably 1 to 15% by weight and more particularly 1 to 10% by weight of a surface-active substance or a mixture of surface-active substances.

12. A cosmetic composition as claimed in any of claims 1 to 11, **characterized in that** it additionally contains at least one antiperspirant and/or deodorant active principle.

13. The use of an oligo-α-olefin obtainable by oligomerization of
(a) at least one branched α-olefin containing 5 to 18 carbon atoms,
(b) at least one linear α-olefin containing 4 to 10 carbon atoms,
(c) a mixture of a branched α-olefin containing 4 to 18 carbon atoms and a linear α-olefin containing 3 to 18 carbon atoms or
(d) a mixture of various branched α-olefins containing 4 to 18 carbon atoms and linear α-olefins containing 3 to 18 carbon atoms
in the presence of a catalyst selected from the group consisting of organic acids, cationic ion exchangers, silica gels, layer silicates, inorganic acids or Lewis-acid-based catalysts and, optionally, subsequent hydrogenation of the oligomerization product,
as oil components in cosmetic or pharmaceutical preparations, more particularly in antiperspirant and/or deodorant formulations.

## Revendications

1. Composition cosmétique contenant au moins une oligo-α-oléfine ramifiée,
**caractérisée en ce que**
les chaînes latérales au niveau d'au moins un point de ramification sont des radicaux éthyle, propyle ou des radicaux alkyle ramifiés à chaîne plus longue, l'oligo-α-oléfine ramifiée pouvant être obtenue par oligomérisation
a) d'au moins une α-oléfine ramifiée ayant de 5 à 18 atomes de carbone,
b) d'au moins une α-oléfine linéaire ayant de 4 à 10 atomes de carbone,
c) d'un mélange d'une α-oléfine ramifiée ayant de 4 à 18 atomes de carbone et d'une α-oléfine linéaire ayant 3 à 18 atomes de carbone ou
d) d'un mélange de différentes α-oléfines ramifiées ayant de 4 à 18 atomes de carbone et d'α-oléfines linéaires ayant de 3 à 18 atomes de carbone,
en présence d'un catalyseur choisi dans le groupe constitué des acides organiques, des échangeurs d'ions cationiques, des gels de silice, des silicates lamellaires, des acides inorganiques ou des catalyseurs à base d'acide de Lewis.

2. Composition cosmétique selon la revendication 1,
**caractérisée en ce que**
l'oligo-α-oléfine ramifiée comporte au total 12 à 36, de préférence 12 à 24 et notamment 14 à 24 atomes de carbone.

3. Composition cosmétique selon la revendication 1 ou 2,
**caractérisée en ce que**
l'oligo-α-oléfine ramifiée issue de l'oligomérisation est ensuite hydrogénée.

4. Composition cosmétique selon l'une au moins des revendications 1 à 3,
**caractérisée en ce qu'**
on oligomérise un mélange (c) composé d'une α-oléfine ramifiée ayant de 5 à 12 atomes de carbone et d'une α-oléfine linéaire ayant de 3 à 12 atomes de carbone en présence d'un catalyseur choisi dans le groupe constitué des acides organiques, des échangeurs d'ions cationiques, des gels de silice, des silicates lamellaires, des acides inorganiques ou des catalyseurs à base d'acide de Lewis.

5. Composition cosmétique selon l'une au moins des revendications 1 à 4,
**caractérisée en ce que**
l'α-oléfine linéaire est choisie dans le groupe constitué du 1-propène, 1-butène, 2-butène, 1-pentène et 2-pentène.

6. Composition cosmétique selon l'une au moins des revendications 1 à 5,
**caractérisée en ce que**
l'α-oléfine ramifiée est choisie dans le groupe constitué du 2-éthyl-1-hexène, 2-propylheptène, 2-méthyl-1-butène, 2-méthyl-1-pentène, 3-méthyl-1-pentène ou 4-méthyl-1-pentène.

7. Composition cosmétique selon l'une au moins des revendications 1 à 6,
**caractérisée en ce qu'**
on oligomérise un mélange composé de 80 % de butène et de 20 % d'isobutène en présence d'un catalyseur choisi dans le groupe constitué des acides organiques, des échangeurs d'ions cationiques, des gels de silice, des silicates lamellaires, des acides inorganiques ou des catalyseurs à base d'acide de Lewis.

8. Composition cosmétique selon l'une au moins des revendications 1 à 7,
**caractérisée en ce qu'**
elle se présente sous la forme une émulsion E/H ou H/E.

9. Composition cosmétique selon l'une au moins des revendications 1 à 8,
**caractérisée en ce qu'**
elle contient de 1 à 50 % en poids, de préférence de 5 à 40 % en poids et notamment de 5 à 25 % en poids de corps huileux.

10. Composition cosmétique selon l'une des revendications 1 à 9,
**caractérisée en ce que**
la proportion de la ou des oligo-α-oléfine(s) par rapport à la quantité totale des corps huileux va de 0,1 à 100 % en poids, de préférence de 1 à 50 % en poids et tout particulièrement de 1 à 20 % en poids.

11. Composition cosmétique selon l'une des revendications 1 à 10,
**caractérisée en ce qu'**
elle contient de 0,1 à 20 % en poids, de préférence de 1 et 15 % en poids et notamment de 1 et 10 % en poids d'une substance tensioactive ou d'un mélange de substances tensioactives.

12. Composition cosmétique selon l'une des revendications 1 à 11,
**caractérisée en ce qu'**
elle contient en outre au moins une substance active anti-transpirante et/ou déodorante.

13. Utilisation d'oligo-α-oléfine pouvant être obtenue par oligomérisation, puis éventuellement par hydrogènation
(a) d'au moins une α-oléfine ramifiée ayant de 5 à 18 atomes de carbone,
(b) d'au moins une α-oléfine linéaire ayant de 4 à 10 atomes de carbone,
(c) d'un mélange d'une α-oléfine ramifiée ayant de 4 à 18 atomes de carbone et d'une α-oléfine linéaire ayant de 3 à 18 atomes de carbone ou
(d) d'un mélange de différentes oléfines ramifiées ayant de 4 à 18 atomes de carbone et α-oléfines linéaires ayant de 3 à 18 atomes de carbone,
en présence d'un catalyseur choisi dans le groupe constitué des acides organiques, des échangeurs d'ions cationiques, des gels de silice, des silicates lamellaires, des acides inorganiques ou des catalyseurs à base d'acide de Lewis,
en tant que corps huileux dans des préparations cosmétiques ou pharmaceutiques, notamment dans des formulations anti-transpirantes et/ou déodorantes.
